# NEUE EUROPÄISCHE PATENTSCHRIFT

(11) **EP 1 817 529 B2**
(45) Veröffentlichungstag und Bekanntmachung des Hinweises auf die Entscheidung über den Einspruch: **08.01.2014**
(45) Hinweis auf die Patenterteilung: 16.03.2011
(21) Anmeldenummer: 05799138.2
(22) Anmeldetag: 25.10.2005
(51) Int. Cl.: F24D 3/12

(54) **EINRICHTUNG UND VERFAHREN ZUR ERFASSUNG VON UMGEBUNGSPARAMETERN BEI FUßBODENBELÄGEN UND FUßBODENBELAG MIT EINER SOLCHEN EINRICHTUNG**
DEVICE AND METHOD FOR DETECTING ENVIRONMENTAL PARAMETERS FOR FLOOR COVERINGS AND A FLOOR COVERING ASSOCIATED WITH A DEVICE OF THIS TYPE
PROCEDE ET DISPOSITIF POUR SAISIR DES PARAMETRES CONCERNANT L'ENVIRONNEMENT DE REVETEMENTS DE SOL ET REVETEMENT DE SOL DOTE D'UN DISPOSITIF DE CE TYPE

(30) Priorität: 30.11.2004 AT 20092004
(43) Veröffentlichungstag der Anmeldung: 15.08.2007
(73) Patentinhaber: Jilg, Helmut, 3051 St. Christophen (AT); Jilg, Josef, 3051 St. Christrophen (AT)
(72) Erfinder: Jilg, Helmut, 3051 St. Christophen (AT); Jilg, Josef, 3051 St. Christrophen (AT)
(74) Vertreter: Sonn & Partner Patentanwälte
(86) Internationale Anmeldenummer: PCT/AT2005/000422
(87) Internationale Veröffentlichungsnummer: WO 2006/058350

(56) Entgegenhaltungen:
- EP-A- 0 530 581
- EP-A1- 0 918 212
- DE-A- 1 258 014
- DE-A1- 1 579 777
- DE-A1- 2 506 363
- DE-A1- 19 913 931
- DE-C3- 3 922 556
- DE-U1- 9 210 128
- DE-U1- 29 814 738
- US-A- 5 711 480
- US-A1- 2002 060 252
- US-A1- 2004 046 039

## Beschreibung

Die Erfindung betrifft die Verwendung einer Fußbodenbelags-Erfassungseinrichtung zur Erfassung von Umgebungsparametern der Fußbodenbeläge mit zumindest einem Sensor zur Messung der Temperatur im Bereich des Fußbodenbelags.

Die Erfindung betrifft weiters einen Fußbodenbelag bestehend aus Fußbodenbelagselementen zur Auflage auf einen Estrich.

Schließlich betrifft die Erfindung ein Verfahren zur Erfassung von Umgebungsparametern bei Fußbodenbelägen, wobei die Temperatur im Bereich des Fußbodenbelags gemessen wird.

Fußbodenbeläge sind einer ständigen Abnützung und den Umgebungseinflüssen, insbesondere der Temperatur und Luftfeuchtigkeit, ausgesetzt, welche deren Lebensdauer stark beeinflussen. Häufig kommt es bei Schäden an Fußbodenbelägen zu Reklamationen, meist an den Fußbodenleger. Viele Schäden an den Fußbodenbelägen treten durch Einflüsse auf, welche der Fußbodenleger nicht zu vertreten hat. Beispielsweise treten bei Holzfußböden häufig folgende Mängel auf:
- Große Fugen (Spaltenbildung)
- Holzbodenelementablösungen
- Trocknungsschüsselung oder durch feuchte Zunahme oder Abnahme ausgelöste Schüsselung der Elemente
- Nutschichtablösungen bei Fertigpaketten.

Diese genannten Mängel treten insbesondere bei Holzfußböden mit Fußbodenheizung häufig auf und sind auf zu hohe Vorlauftemperaturen der Fußbodenheizung mit der daraus resultierenden geringen Luftfeuchtigkeit zurückzuführen. Die Ursache für die entstehenden Mängel ist jedoch in vielen Fällen nicht eindeutig zu eruieren oder erfordert die Erstellung aufwendiger Sachverständigen-Gutachten. Häufig wird der Fußbodenleger zu Unrecht zu einer Behebung des Mangels verpflichtet.

Die vorliegende Erfindung ist jedoch nicht auf Holzfußböden beschränkt, sondern kann prinzipiell auf alle Arten von Fußböden, wie z.B. Textil, PVC, Stein oder Keramik angewendet werden. Schließlich kommt es durch ein Zusammenspiel verschiedener Professionisten auf den Baustellen häufig zu einem ungenügenden oder teilweise sogar falschen Informationsaustausch der ebenfalls zu frühzeitigen Mängeln an den Fußbodenbelägen führen kann. Üblicherweise muss der Handwerker die Vorarbeiten des anderen Handwerkers prüfen und übernehmen. Dies wird durch ständig wechselnde Rezepturen oder chemischen Zusammensetzungen verwendeter Stoffe besonders erschwert.

Es sind daher Bestrebungen im Gange, den Informationsaustausch bei Estrich- und Bodenbelägsarbeiten zu normieren. Beispielsweise wurde ein so genannter Fußbodenpass ausgearbeitet, der eine detaillierte Dokumentation über die gesamte Fußbodenkonstruktion einschließlich Bodenbelag umfasst. Die Dokumentation bringt jedoch nur dann einen Gewinn, wenn sie verfügbar ist. Häufig werden die Dokumentationen zwar bei der Verlegung des Fußbodens übergeben, sind jedoch nach vielen Jahren, wenn die Mängel auftreten, nicht mehr auffindbar.

Für Renovierungsarbeiten an Fußbodenbelägen wären Informationen, welcher Estrich verwendet wurde, welche Zusatzstoffe beigemischt wurden, welcher Voranstrich, Spachtelmasse und Klebstoffe verwendet wurden, sowie Daten der Hersteller der verwendeten Materialien, usw. extrem hilfreich. Diese Informationen würden auch bei späteren Reklamationen die Klärung der Haftungsfrage erleichtern.

Zu Zwecken der Hinterlegung wichtiger Daten eines Fahrzeuges beschreibt beispielsweise die US 6 275 157 B1 ein Kraftfahrzeug, welches einen Transponder in der Windschutzscheibe enthält, in dem wichtige Daten über das Fahrzeug gespeichert sind, welche bei Bedarf ausgelesen werden können.

Zur Überwachung der Überschreitung bestimmter oberer Temperaturgrenzen ist es bekannt, zwischen einem Holzfußboden und dem Estrich eine Plombe aus Metall anzuordnen, welche sich bei Überschreitung der Temperatur über den Grenzwert verfärbt. Zur Klärung, ob die Vorlauftemperatur der Fußbodenheizung zu hoch war, muss jedoch der Fußboden geöffnet werden, um zur Plombe zu gelangen.

Die US 2002/0060252 A1 betrifft ein Heizungssystem mit zwei verschiedenen Heizeinrichtungen, die zur Erhöhung des Komfortgefühls der Person speziell geregelt werden, indem eine Kopplung der Heizeinrichtungen untereinander vorgenommen wird. Dabei existieren Temperatursensoren zur Messung der Raumtemperatur und auch zur Messung der Fußbodentemperatur.

Die EP 530 581 A betrifft eine Temperaturregelung von Fußbodenheizsystemen, durch welche Schäden an Bodenbelägen vermieden werden sollen. Dabei wird sowohl die Messung der Temperatur als auch die Messung von Feuchtigkeitswerten erwähnt, ohne Hinweis darauf wo diese Parameter ermittelt werden.

Die US 2001/0044588 A1 beschreibt eine Erfassungseinrichtung zur Messung physiologischer Parameter wie z.B. der Temperatur und Übertragung der erfassten Parameter an Einrichtungen wie z.B. den Computer des behandelten Arztes.

Die DE 39 22 556 C3 betrifft eine Anordnung zur kontaktlosen Energie - und Sensorsignalübertragung. Aus der US 5,711,480 A ist eine Gebäude-Klima-anlagen-Regelung bekannt geworden. Schließlich zeigt die DE 25 06 363 eine Steuerung einer Warmwasser-Fußbodenheizung

Die Aufgabe der vorliegenden Erfindung besteht in der Schaffung einer oben erwähnten Verwendung einer Fußbodenbelags-Erfassungseinrichtung, durch die Umgebungsparameter der Fußbodenbeläge erfasst werden können, um die Ursache allfälliger Schäden bei späteren Reklamationen mit größerer Sicherheit ermitteln zu können. Darüber hinaus soll Information, die für die Ermittlung von Schadensursachen relevant ist, bereitgestellt werden.

Die weitere Aufgabe der vorliegenden Erfindung besteht in der Schaffung eines Fußbodenbelags, der die Information, welche für die Ermittlung der Ursache von Schäden am Fußboden relevant ist, bereitstellt.

Schließlich liegt eine weitere Aufgabe der Erfindung in der Schaffung eines Verfahrens zur Erfassung von Umgebungsparametern bei Fußbodenbelägen, durch das für das Auftreten von Schäden am Fußboden relevante Parameter ermittelt werden können und somit die Ursache der Schäden eindeutig geklärt werden kann.

Die erste erfindungsgemäße Aufgabe wird durch die oben genannte Verwendung einer Fußbodenbelags-Erfassungseinrichtung gelöst, wobei der zumindest eine Temperatursensor zur Anbringung unterhalb des Fußbodenbelags vorgesehen ist, und mit einer Einrichtung zur drahtlosen Übertragung der gemessenen Temperaturwerte zu einem Empfänger verbunden ist, welche Übertragungseinrichtung durch einen Transponder gebildet ist und auch zum Empfang von Daten ausgebildet ist. Durch die Messung der Temperatur unterhalb des Fußbodenbelags und die drahtlose Übermittlung der gemessenen Temperaturwerte zu einem Empfänger ist eine ständige Überwachung der Temperatur ohne Zerstörung des Fußbodenbelags möglich. Die Überwachung der Temperatur des Fußbodens ist insbesondere bei Vorhandensein von Fußbodenheizungen, welche häufig eine zu hohe Vorlauftemperatur aufweisen, von hoher Bedeutung. Dabei wird die Temperatur an den kritischen Stellen, nämlich zwischen Fußbodenbelag und Estrich gemessen. Diese Temperatur ist für die Alterung des Fußbodens entscheidend. Die Erfindung ist jedoch nicht auf Holzfußbodenbeläge beschränkt, sondern kann beispielsweise auch bei Keramikbelägen bedeutsam sein. Beispielsweise kann mit der gegenständlichen Erfassungseinrichtung auch die Temperatur bei Fliesen registriert werden und dadurch die Ursache für allfällige Frostschäden dokumentiert werden. Durch die Ausbildung der Einrichtung zur drahtlosen Übertragung durch einen Transponder können an die unterhalb des Fußbodenbelags angeordnete Elektronik auch Daten oder Signale übermittelt werden, welche beispielsweise die Temperaturmessung aktivieren oder andere Prozesse auslösen können.

Dabei ist die Übertragungseinrichtung vorzugsweise durch ein Hochfrequenzidentifikations(RFID)-System gebildet. Derartige RFID(Radio Frequency Identification)-Systeme sind für die eindeutige Kennzeichnung verschiedenster Produkte üblich. Derartige RFID-Systeme sind extrem klein und können somit einfach zwischen dem Fußbodenbelag und dem Estrich angeordnet werden. Darüber hinaus sind die Herstellungskosten derartiger RFID-Systeme sehr gering, so dass eine breite Anwendung sichergestellt werden kann. Dabei ist das Hochfrequenzidentifikations(RFID)-System vorzugsweise passiv, d.h. ohne eigene Spannungsversorgung ausgebildet. Bei der Übertragung der gemessenen Temperaturwerte oder anderer Daten wird die dafür notwendige Energie von außen in das RFID-System durch ein elektromagnetisches Feld eingebracht.

Um das Speichern relevanter Daten über den Fußbodenbelag oder gemessener Werte zu ermöglichen, kann ein mit der Übertragungseinrichtung verbundener Speicher vorgesehen sein.

Dabei kann ein Speicher durch einen reinen Lesespeicher (ROM Read Only Memory) gebildet sein.

Dieser Lesespeicher kann eine eindeutige Identifikation, wie sie bei den RFID-Systemen üblich sind, enthalten, welche eine eindeutige Zuordnung zwischen Fußbodenbelag, und beispielsweise dessen Hersteller oder dgl. erlaubt.

Darüber hinaus kann der Speicher durch einen Lese-/Schreibspeicher (RWM Read Write Memory) gebildet sein und somit auch das Ablegen beispielsweise gemessener Werte von Umgebungsparametern oder auch von außen eingebrachter Informationen ermöglichen.

Gemäß einem weiteren Merkmal der Erfindung ist zumindest ein mit der Übertragungseinrichtung verbundener Sensor zur Messung der Feuchtigkeit vorgesehen. Insbesondere bei Holzfußböden ist die Feuchtigkeit für den Alterungsprozess auch besonders relevant.

Weiters kann beispielsweise zur Ermittlung der Beanspruchung eines Fußbodenbelags das Vorsehen eines mit der Übertragungseinrichtung verbundenen Drucksensors wichtige Information liefern. Der Drucksensor kann beispielsweise durch ein piezoelektrisches Element gebildet sein und einen über eine gewisse Zeitspanne gemittelten Messwert liefern.

Wenn ein Temperatursensor und allenfalls ein Feuchtigkeitssensor zur Messung der Temperatur und allenfalls Feuchtigkeit an der Oberfläche des Fußbodenbelags ausgebildet ist, kann weitere wichtige Information über die Umgebungsparameter des Fußbodenbelags ermittelt werden. Häufig ist auch der Unterschied zwischen der Temperatur und Feuchtigkeit oberhalb des Fußbodenbelags und der Temperatur und Feuchtigkeit unterhalb des Fußbodenbelags für bestimmte Schäden verantwortlich und somit deren Kenntnis besonders wichtig. Um die Messung der Temperatur und allenfalls Feuchtigkeit an der Oberfläche des Fußbodenbelags zu ermöglichen, kann beispielsweise eine Bohrung mit möglichst geringem Durchmesser im Fußbodenbelag vorgesehen sein, in der der Temperatur- und allenfalls Feuchtigkeitssensor angeordnet ist.

Weiters kann eine mit der Übertragungseinrichtung verbundene Spannungsversorgung, insbesondere eine Batterie, vorgesehen sein. Diese Spannungsversorgung begrenzt zwar die Lebensdauer der Erfassungseinrichtung, kann dafür eine höhere Reichweite der gesendeten Messdaten oder hinterlegten Daten mit sich bringen. Durch entsprechende Wahl der Spannungsversorgung und Auswahl besonders verbrauchsarmer Komponenten kann jedoch eine Lebensdauer von einigen Jahren erzielt werden.

Die Übertragungseinrichtung, der zumindest eine Temperatursensor, allenfalls der Feuchtigkeitssensor, allenfalls der zumindest eine Drucksensor sowie der zumindest eine Speicher können durch eine gemeinsame integrierte Schaltung gebildet werden. Diese ist bei entsprechender Stückzahl besonders kostengünstig herstellbar und kann besonders rasch und einfach auf die Unterseite der Fußbodenbeläge angebracht werden.

Die zweite erfindungsgemäße Aufgabe wird durch einen oben genannten Fußbodenbelag gelöst, wobei an der Unterseite zumindest eines den Fußbodenbelag bildenden Elements eine oben erwähnte Erfassungseinrichtung angeordnet ist. Somit kann bei einem derartigen Fußbodenbelag die Ursache später auftretender Schäden besser ergründet werden und die Schuldfrage bei Reklamationen schneller und vor allem sicherer geklärt werden.

Dabei kann die Erfassungseinrichtung in einer Aussparung an der Unterseite eines Fußbodenbelagselements angeordnet sein. Bei einem Holzfußboden kann eine derartige Aussparung durch Fräsen besonders leicht hergestellt werden.

Die Erfassungseinrichtung kann mit der Unterseite des Fußbodenbelagselements verklebt sein. Dabei kann die Verklebung derart vorgenommen werden, dass eine Ablösung der Erfassungseinrichtung unweigerlich zur Zerstörung derselben führt. Somit ist eine nachträgliche Manipulation an der Erfassungseinrichtung ausgeschlossen.

Alternativ dazu kann die Erfassungeinrichtung auch lösbar mit der Unterseite des Fußbodenbelagselements verbunden, beispielsweise verschraubt, sein. Auch bei einer derartigen Verbindungsart kann die Manipulation beispielsweise durch Plomben oder Lackierungen zumindest sichtbar gemacht werden.

Gemäß einem weiteren Merkmal der Erfindung sind die Fußbodenbelagselemente aus Holz gebildet.

Zumindest ein Element des Fußbodenbelags kann eine Bohrung zur Aufnahme eines Sensors zur Messung der Temperatur und allenfalls zur Aufnahme eines Sensors zur Messung der Feuchtigkeit an der Oberfläche des Fußbodenbelags aufweisen. Dadurch kann auch die Temperatur und allenfalls Feuchtigkeit an der Oberfläche des Fußbodenbelags ermittelt und drahtlos an einen Empfänger weitergeleitet werden.

Gelöst wird die weitere erfindungsgemäße Aufgabe auch durch ein oben genanntes Verfahren zur Erfassung von Umgebungsparametern bei Fußbodenbelägen, wobei die an der Unterseite des Fußbodenbelags gemessenen Temperaturwerte an eine als Transponder auch zum Empfang von Daten ausgebildete Einrichtung zur drahtlosen Übertragung der gemessenen Temperaturwerte weitergeleitet und auf Anforderung drahtlos von der Übertragungseinrichtung an einen Empfänger übermittelt werden.

Weiters können an der Unterseite des Fußbodenbelags gemessene Feuchtigkeitswerte und allenfalls Druckwerte drahtlos an den Empfänger übermittelt werden.

Schließlich können auch an der Oberseite des Fußbodenbelags gemessene Temperatur- und allenfalls Feuchtigkeitswerte drahtlos an den Empfänger übermittelt werden.

Weiters können dem Fußbodenbelag zugeordnete Daten drahtlos an den Empfänger übermittelt werden.

Dabei können die Temperaturwerte, allenfalls die Feuchtigkeitswerte, allenfalls die Druckwerte und allenfalls die zugeordneten Daten automatisch zu vorgegebenen Zeiten an den Empfänger übermittelt werden. Dies erfordert jedoch das Vorhandensein einer Spannungsversorgung, welche die entsprechende Schaltung zu den vorgegebenen Zeiten aktiviert und die Daten an den Empfänger sendet.

Ebenso können die Temperaturwerte, allenfalls die Feuchtigkeitswerte, allenfalls die Druckwerte und allenfalls die zugeordneten Daten auch auf Anforderung an den Empfänger übermittelt werden. Auf diese Weise kann bei einem passiven Hochfrequenzidentifikations-System durch entsprechendes Anordnen des Empfängers über der Übertragungseinrichtung die für die Messung der Umgebungsparameter und die Übertragung der gemessenen Werte erforderliche Energie über ein elektromagnetisches Feld eingebracht werden.

Schließlich können die Temperaturwerte, allenfalls die Feuchtigkeitswerte, allenfalls die Druckwerte und allenfalls die zugeordneten Daten auch automatisch zu vorgegebenen Zeiten vor der Übertragung an den Empfänger in einem Speicher zwischengespeichert werden. Der Speicher kann zu vorgegebenen Zeiten automatisch seine Werte drahtlos an einen Empfänger übermitteln oder auch seinen Inhalt bei Aufforderung an einen Empfänger senden.

Um einen Missbrauch zu verhindern, können die Temperaturwerte, allenfalls die Feuchtigkeitswerte, allenfalls die Druckwerte und allenfalls die zugeordneten Daten vor der Übertragung an den Empfänger verschlüsselt werden.

Gemäß einem weiteren Merkmal ist vorgesehen, dass die Temperaturwerte und allenfalls die Feuchtigkeitswerte zur Regelung der Temperatur einer unter dem Fußbodenbelag angeordneten Fußbodenheizung herangezogen werden. Auf diese Weise könnten zu hohe, für den Fußbodenbelag schädliche Vorlauftemperaturen der Fußbodenheizung verhindert und somit eine höhere Lebensdauer des Fußbodenbelags erzielt werden. Bei entsprechender Reichweite der Übertragungseinrichtung kann eine Übertragung der relevanten Temperatur- und allenfalls Feuchtigkeitswerte über mehrere Meter zur Regeleinrichtung der Heizung erfolgen, wodurch der Installationsaufwand sehr gering gehalten werden kann.

Die vorliegende Erfindung wird anhand der beigefügten Zeichnungen näher erläutert.

Darin zeigen:
- Fig. 1: ein Blockschaltbild einer Ausführungsvariante einer Fußbo- denbelags-Erfassungseinrichtung;
- Fig. 2: ein Blockschaltbild einer weiteren Ausführungsform einer erfindungsgemäßen Fußbodenbelags-Erfassungseinrichtung;
- Fig. 3: eine perspektivische Ansicht zur Veranschaulichung der An- wendung der gegenständlichen Erfindung;
- Fig. 4: ein Schnittbild durch einen Fußbodenbelag zur Veranschau- lichung der Anordnung einer Fußbodenbelags-Erfassungsein- richtung an der Unterseite des Fußbodenbelags; und
- Fig. 5: ein schematisches Blockschaltbild zur Veranschaulichung der Regelung einer Fußbodenheizung mit Hilfe der erfin- dungsgemäßen Fußbodenbelags-Erfassungseinrichtung.

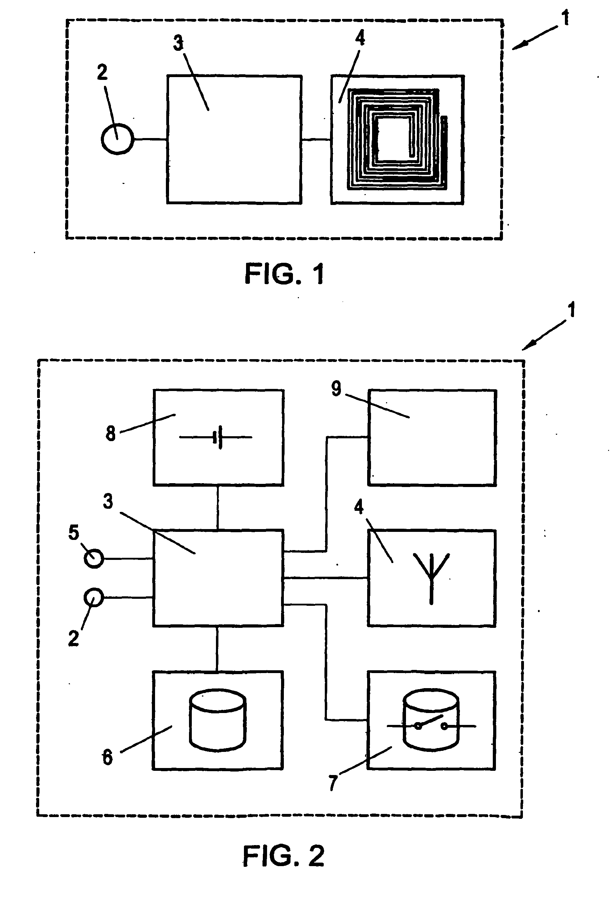

Fig. 1 zeigt eine Fußbodenbelags-Erfassungseinrichtung 1 zur Erfassung von Umgebungsparametern, beispielsweise Temperaturen der Fußbodenbeläge, mit einem Sensor 2 zur Messung der Temperatur im Bereich des Fußbodenbelags. Der Sensor 2 ist mit einer Einrichtung 3 zur drahtlosen Übertragung der gemessenen Temperaturwerte zu einem Empfänger verbunden. Zur drahtlosen Übertragung ist eine entsprechende Antenne 4 vorgesehen. Vorteilhafterweise ist die Fußbodenbelags-Erfassungseinrichtung 1 durch ein so genanntes Hochfrequenzidentifikations(RFID)-System gebildet. Derartige Transponder können besonders klein und kostengünstig hergestellt werden. Je nach verwendeter Frequenz für die Übertragung sind verschiedene Reichweiten für die Übertragung der ermittelten Daten erzielbar.

Fig. 2 zeigt das Blockschaltbild einer weiteren Ausführungsvariante der Fußbodenbelags-Erfassungseinrichtung 1 zur Erfassung von Umgebungsparametern bei Fußbodenbelägen, wobei neben dem Sensor 2 zur Messung der Temperatur auch ein Sensor 5 zur Messung der Feuchtigkeit an der Unterseite des Fußbodenbelags vorgesehen ist. Die Sensoren 2, 5 sind mit der Einrichtung 3 zur drahtlosen Übertragung der gemessenen Sensorwerte verbunden. Weiters ist mit der Übertragungseinrichtung 3 ein Speicher 6 verbunden, der durch einen Lesespeicher (ROM - Read Only Memory) gebildet ist und eine eindeutige Identifikation enthält. Somit kann eine eindeutige Zuordnung zum Fußbodenbelag hergestellt werden. Neben der elektronischen Verankerung der Identifikation ist natürlich auch eine beispielsweise durch entsprechende Schalter gebildete Kodierung denkbar.

Neben dem Speicher 6 kann auch ein durch einen Lese-/Schreibspeicher gebildeter Speicher 7 vorgesehen sein, in den beispielsweise die ermittelten Werte der Sensoren 2, 5 geschrieben werden können. Daneben kann der Speicher 7 auch Daten über den Estrich, den Fußbodenbelag, den Hersteller des Fußbodenbelags, den Verleger und vieles mehr enthalten. Ein Schreiben von Daten von Außen in den Speicher 7 kann ebenfalls ermöglicht werden. Für ein aktives System kann eine Spannungsversorgung 8, welche beispielsweise durch eine Batterie gebildet ist, vorgesehen sein. Schließlich kann ein Zeitmodul 9 vorgesehen sein, welches eine zeitliche Zuordnung der gemessenen Werte erlaubt.

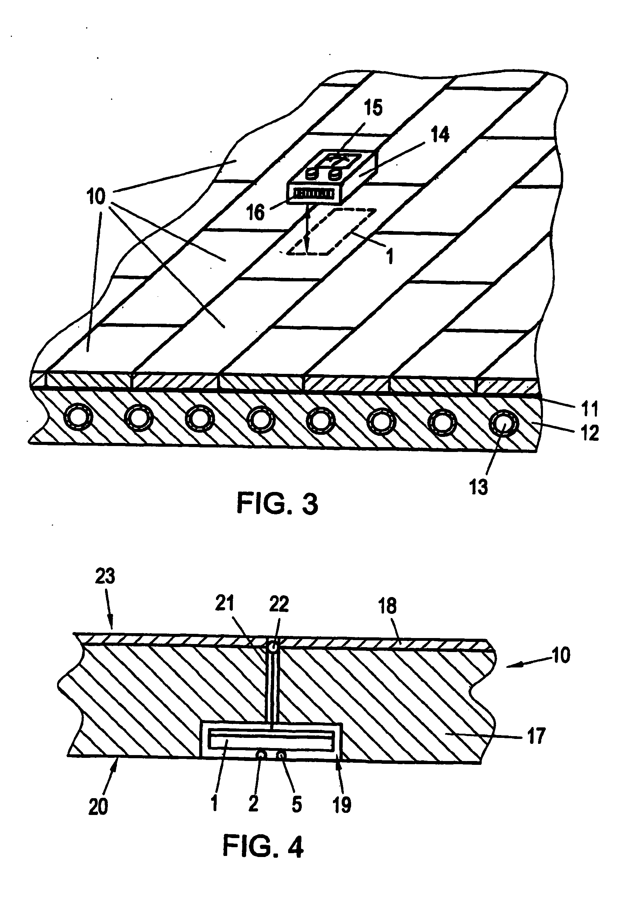

Fig. 3 zeigt ein perspektivisches, teilweise geschnittenes Bild eines Fußbodenbelags bestehend aus mehreren Elementen 10, beispielsweise Holzelementen, welche über eine entsprechende Spachtelmasse 11 auf einen Estrich 12 aufgelegt werden. Im Estrich 12 sind die Heizungsrohre für eine Fußbodenheizung angeordnet. Die erfindungsgemäße Verwender Fußbodenbelags-Erfassungseinrichtung 1 zur Erfassung von Umgebungsparametern bei Fußbodenbelägen ist an der Unterseite eines Fußbodenbelagselements 10 angeordnet. Ein Empfänger 14 kann durch entsprechendes Anordnen über der Fußbodenbelags-Erfassungseinrichtung 1 die gemessenen Temperaturwerte aber auch Daten über den Fußbodenbelag empfangen und beispielsweise auf einer Anzeige 15 direkt zur Anzeige bringen. Ebenso kann der Empfänger 14 über eine entsprechende Schnittstelle 16 die relevanten Daten beispielsweise an einen Computer (nicht dargestellt) zur weiteren Verarbeitung weiterleiten.

Fig. 4 zeigt ein Schnittbild durch ein mit einer Fußbodenbelags-Erfassungseinrichtung 1 ausgestatteten Fußbodenbelagselement 10. Im dargestellten Beispiel handelt es sich beim Element 10 um ein Holzelement. Dabei ist auf einer Holzschicht 17 eine Nutzschicht 18 aus ensprechendem Holzmaterial angeordnet. Die erfindungsgemäße Fußbodenbelags-Erfassungseinrichtung 1 ist in einer Aussparung 19 an der Unterseite 20 des Elements 10 platziert. Mit der Fußbodenbelags-Erfassungseinrichtung 1 sind ein Temperatursensor 2 und allenfalls ein Feuchtigkeitssensor 5 verbunden bzw. darin integriert. Der Temperatursensor 2 ermittelt die Temperatur an der Unterseite des Fußbodenbelagselements 10. Der Feuchtigkeitssensor 5 ermittelt die Feuchtigkeit an der Unterseite 20 des Fußbodenbelagselements 10. Weiters kann im Element 10 eine Bohrung 21 angeordnet sein, in der ein Sensor 22 zur Messung der Temperatur und allenfalls Feuchtigkeit an der Oberfläche 23 des Elements 10 enthalten ist. Somit ist es möglich, die relevanten Umgebungsparameter zu erfassen und drahtlos an einen Empfänger 14 weiterzuleiten. Weiters können jederzeit relevante Daten über den Fußbodenbelag, welche in einem entsprechenden Speicher 7 der Erfassungseinrichtung 1 enthalten sind, abgerufen werden.

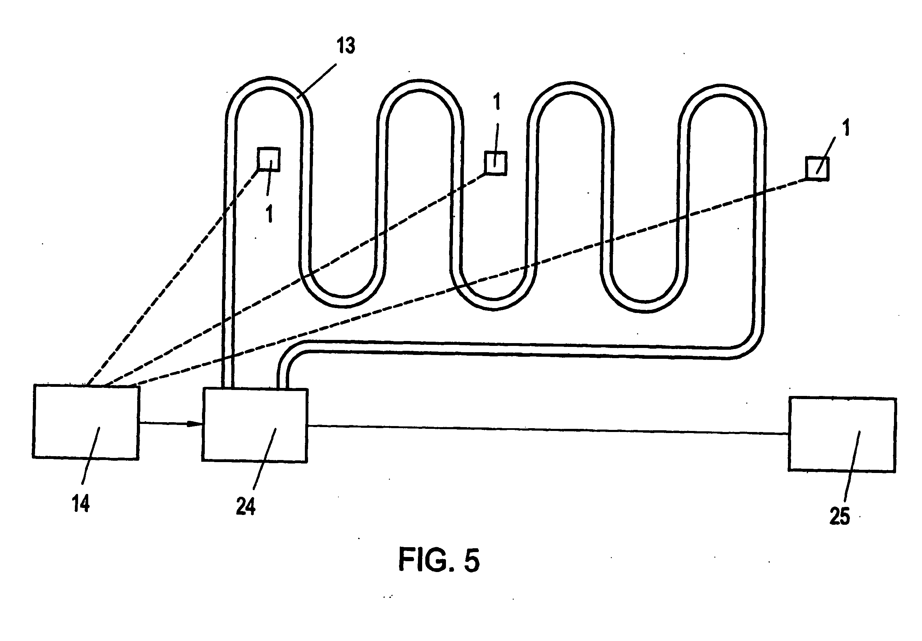

Schließlich zeigt Fig. 5 ein schematisches Blockschaltbild einer Fußbodenheizung bestehend aus einer Heizungssteuerung 24 und Heizungsrohren 13, welche im Estrich eingebettet sind. Über den Heizungsrohren 13 ist der Fußbodenbelag (nicht dargestellt) angeordnet. An bestimmten Stellen des Fußbodens sind Fußbodenbelags-Erfassungseinrichtungen 1 zur Erfassung von Umgebungsparametern der Fußbodenbeläge angeordnet. Die Fußbodenbelags-Erfassungseinrichtungen 1 senden automatisch, vorzugsweise zu bestimmten Zeiten, ihre Daten an einen Empfänger 14, der mit der Heizungssteuerung 24 verbunden ist. Somit kann die Vorlauftemperatur der durch die Heizungsrohre 13 fließenden Flüssigkeit so geregelt werden, dass die Temperatur unterhalb des Fußbodenbelags bestimmte Grenzwerte nicht überschreitet. Somit kann eine hohe Lebensdauer des Fußbodenbelags erzielt werden. Im Normalfall ist die Heizungssteuerung 24 mit einem Zimmerthermostat 25 verbunden und die Vorlauftemperatur der Fußbodenheizung von der Temperatur dieses Zimmer- oder Außenthermostats abhängig. Im Falle einer niedrigen Zimmer- bzw. Außentemperatur wird in der Folge mit einer zu hohen Vorlauftemperatur der Fußboden beheizt, was zu vorzeitigen Schäden am Fußbodenbelag führt.

Durch die vorliegende Erfindung ist es möglich, auch sensiblere Holzarten (beispielsweise Kumaru, Ipe, kanadische Ahorn oder dgl.) für Fußbodenbeläge zu verwenden, welche derzeit aufgrund der hohen Wahrscheinlichkeit einer Fugenbildung, insbesondere durch Fußbodenheizungen, nicht verwendbar sind.

Die vorliegende Erfindung dient dazu, die Umgebungsparameter bei Fußbodenbelägen, insbesondere für die Klärung der Schadensursachen, zu ermitteln und andererseits relevante Daten über den Fußbodenbelag immer verfügbar zu machen. Somit kann ein lückenloser Informationsaustausch zwischen dem Hersteller des Fußbodenbelags, dem Hersteller des Estrichs, dem Fußbodenverleger bis hin zum Handwerker, der eine allfällige Reparatur an dem Fußbodenbelag vornehmen soll, erzielt werden.

## Patentansprüche

1. Verwendung einer Fußbodenbelags-Erfassungseinrichtung (1) zur Erfassung von Umgebungsparametern der Fußbodenbeläge mit zumindest einem Sensor (2) zur Messung der Temperatur im Bereich des Fußbodenbelags, **dadurch gekennzeichnet, dass** der zumindest eine Temperatursensor (2) zur Anbringung zwischen dem Fußbodenbelag und einem Estrich vorgesehen ist, und mit einer Einrichtung (3) zur drahtlosen Übertragung der gemessenen Temperaturwerte zu einem Empfänger (14) verbunden ist, welche Übertragungseinrichtung (3) durch einen Transponder gebildet ist und auch zum Empfang von Daten ausgebildet ist.

2. Verwendung einer Fußbodenbelags-Erfassungseinrichtung nach Anspruch 1, **dadurch gekennzeichnet, dass** die Übertragungseinrichtung (3) durch ein Hochfrequenzidentifikations(RFID)-System gebildet ist.

3. Verwendung einer Fußbodenbelags-Erfassungseinrichtung nach Anspruch 2, **dadurch gekennzeichnet, dass** das Hochfrequenzidentifikations(RFID)-System passiv ausgeführt ist.

4. Verwendung einer Fußbodenbelags-Erfassungseinrichtung nach einem der Ansprüche 1 bis 3, **dadurch gekennzeichnet, dass** zumindest ein mit der Übertragungseinrichtung (3) verbundener Speicher (6; 7) vorgesehen ist.

5. Verwendung einer Fußbodenbelags-Erfassungseinrichtung nach Anspruch 4, **dadurch gekennzeichnet, dass** ein Speicher (6) durch einen Lesespeicher gebildet ist.

6. Verwendung einer Fußbodenbelags-Erfassungseinrichtung nach Anspruch 5, **dadurch gekennzeichnet, dass** der Lesespeicher (6) eine eindeutige Identifikation enthält.

7. Verwendung einer Fußbodenbelags-Erfassungseinrichtung nach einem der Ansprüche 4 bis 6, **dadurch gekennzeichnet, dass** der Speicher (7) durch einen Lese-/Schreibspeicher gebildet ist.

8. Verwendung einer Fußbodenbelags-Erfassungseinrichtung nach einem der Ansprüche 1 bis 7, **dadurch gekennzeichnet, dass** zumindest ein mit der Übertragungseinrichtung (3) verbundener Sensor (5) zur Messung der Feuchtigkeit vorgesehen ist.

9. Verwendung einer Fußbodenbelags-Erfassungseinrichtung nach einem der Ansprüche 1 bis 8, **dadurch gekennzeichnet, dass** zumindest ein mit der Übertragungseinrichtung (3) verbundener Drucksensor vorgesehen ist.

10. Verwendung einer Fußbodenbelags-Erfassungseinrichtung nach einem der Ansprüche 1 bis 9, **dadurch gekennzeichnet, dass** ein Temperatursensor (22) und allenfalls ein Feuchtigkeitssensor zur Messung der Temperatur und allenfalls Feuchtigkeit an der Oberfläche (23) des Fußbodenbelags ausgebildet ist.

11. Verwendung einer Fußbodenbelags-Erfassungseinrichtung nach einem der Ansprüche 1 bis 10, **dadurch gekennzeichnet, dass** eine mit der Übertragungseinrichtung (3) verbundene Spannungsversorgung (8), insbesondere eine Batterie, vorgesehen ist.

12. Verwendung einer Fußbodenbelags-Erfassungseinrichtung nach einem der Ansprüche 4 bis 11, **dadurch gekennzeichnet, dass** die Übertragungseinrichtung (3), der zumindest eine Temperatursensor (2), allenfalls der Feuchtigkeitssensor (5), allenfalls der zumindest eine Drucksensor, sowie der zumindest eine Speicher (6, 7) durch eine integrierte Schaltung gebildet ist.

13. Fußbodenbelag bestehend aus Fußbodenbelagselementen (10) zur Auflage auf einen Estrich (12), **gekennzeichnet durch** eine an der Unterseite (20) zumindest eines Fußbodenbelagselements (10) angeordneten Fußbodenbelags-Erfassungseinrichtung (1) zur Erfassung von Umgebungsparametern der Fußbodenbeläge mit zumindest einem Sensor (2) zur Messung der Temperatur im Bereich des Fußbodenbelags, wobei der zumindest eine Temperatursensor (2) zur Anbringung unterhalb des Fußbodenbelags vorgesehen ist, und mit einer Einrichtung (3) zur drahtlosen Übertragung der gemessenen Temperaturwerte zu einem Empfänger (14) verbunden ist, welche Übertragungseinrichtung (3) **durch** einen Transponder gebildet ist und auch zum Empfang von Daten ausgebildet ist.

14. Fußbodenbelag nach Anspruch 13, **dadurch gekennzeichnet, dass** die Fußbodenbelags-Erfassungseinrichtung (1) in einer Aussparung (19) an der Unterseite (20) eines Fußbodenbelagselements (10) angeordnet ist.

15. Fußbodenbelag nach Anspruch 14, **dadurch gekennzeichnet, dass** die Fußbodenbelags-Erfassungseinrichtung (1) mit der Unterseite (20) des Fußbodenbelagselements (10) verklebt ist.

16. Fußbodenbelag nach Anspruch 14, **dadurch gekennzeichnet, dass** die Fußbodenbelags-Erfassungseinrichtung (1) mit der Unterseite (20) des Fußbodenbelagselements (10) lösbar verbunden, beispielsweise verschraubt, ist.

17. Fußbodenbelag nach einem der Ansprüche 13 bis 16, **dadurch gekennzeichnet, dass** die Fußbodenbelagselemente (10) aus Holz sind.

18. Fußbodenbelag nach einem der Ansprüche 13 bis 17, **dadurch gekennzeichnet, dass** zuminmdest ein Element (10) eine Bohrung (21) zur Aufnahme eines Sensors (22) zur Messung der Temperatur und allenfalls zur Aufnahme eines Sensors zur Messung der Feuchtigkeit an der Oberseite (23) des Fußbodenbelagselements (10) aufweist.

19. Verfahren zur Erfassung von Umgebungsparametern bei Fußbodenbelägen, wobei die Temperatur im Bereich des Fußbodenbelags gemessen wird, **dadurch gekennzeichnet, dass** die an der Unterseite (20) des Fußbodenbelags, zwischen Fußbodenbelag und Estrich gemessenen Temperaturwerte an eine als Transponder auch zum Empfang von Daten ausgebildete Einrichtung (3) zur drahtlosen Übertragung der gemessenen Temperaturwerte weitergeleitet und auf Anforderung drahtlos von der Übertragungseinrichtung (3) an einen Empfänger (14) übermittelt werden.

20. Verfahren nach Anspruch 19, **dadurch gekennzeichnet, dass** an der Unterseite (20) des Fußbodenbelags gemessene Feuchtigkeitswerte drahtlos an den Empfänger (14) übermittelt werden.

21. Verfahren nach Anspruch 19 oder 20, **dadurch gekennzeichnet, dass** an der Unterseite (20) des Fußbodenbelags gemessene Druckwerte drahtlos an den Empfänger (14) übermittelt werden.

22. Verfahren nach einem der Ansprüche 19 bis 21, **dadurch gekennzeichnet, dass** an der Oberseite (23) des Fußbodenbelags gemessene Temperaturwerte drahtlos an den Empfänger (14) übermittelt werden.

23. Verfahren nach einem der Ansprüche 19 bis 22, **dadurch gekennzeichnet, dass** an der Oberseite (23) des Fußbodenbelags gemessene Feuchtigkeitswerte drahtlos an den Empfänger (14) übermittelt werden.

24. Verfahren nach einem der Ansprüche 19 bis 23, **dadurch gekennzeichnet, dass** dem Fußbodenbelag zugeordnete Daten drahtlos an den Empfänger (14) übermittelt werden.

25. Verfahren nach einem der Ansprüche 19 bis 24, **dadurch gekennzeichnet, dass** die Temperaturwerte, allenfalls die Feuchtigkeitswerte, allenfalls die Druckwerte, und allenfalls die zugeordneten Daten automatisch zu vorgegebenen Zeiten an den Empfänger (14) übermittelt werden.

26. Verfahren nach einem der Ansprüche 19 bis 24, **dadurch gekennzeichnet, dass** die Temperaturwerte, allenfalls die Feuchtigkeitswerte, allenfalls die Druckwerte, und allenfalls die zugeordneten Daten auf Anforderung an den Empfänger (14) übermittelt werden.

27. Verfahren nach einem der Ansprüche 19 bis 26, **dadurch gekennzeichnet, dass** die Temperaturwerte, allenfalls die Feuchtigkeitswerte, allenfalls die Druckwerte, und allenfalls die zugeordneten Daten automatisch zu vorgegebenen Zeiten vor der Übertragung an den Empfänger (14) in einem Speicher (7) zwischengespeichert werden.

28. Verfahren nach einem der Ansprüche 19 bis 27, **dadurch gekennzeichnet, dass** die Temperaturwerte, allenfalls die Feuchtigkeitswerte, allenfalls die Druckwerte, und allenfalls die zugeordneten Daten vor der Übertragung an den Empfänger (14) verschlüsselt werden.

29. Verfahren nach einem der Ansprüche 19 bis 28, **dadurch gekennzeichnet, dass** die Temperaturwerte und allenfalls die Feuchtigkeitswerte zur Regelung der Temperatur einer unter dem Fußbodenbelag angeordneten Fußbodenheizung herangezogen werden.

## Claims

1. A use of a floor cover sensor means (1) for sensing ambiance parameters of the floor covers, comprising at least one sensor (2) for measuring the temperature in the region of the floor cover, **characterized in that** said at least one temperature sensor (2) is provided to be positioned between the floor cover and a floor screed and is connected with means (3) for the wireless transmission of the measured temperature values to a receiver (14), the transmission means (3) being formed by a transponder and being also designed for receiving data.

2. The use of a floor cover sensor means according to claim 1, **characterized in that** the transmission means (3) is formed by a radio frequency identification (RFID) system.

3. The use of a floor cover sensor means according to claim 2, **characterized in that** the radio frequency identification (RFID) system is designed passively.

4. The use of a floor cover sensor means according to any of claims 1 to 3, **characterized in that** at least one memory (6; 7) connected with the transmission means (3) is provided.

5. The use of a floor cover sensor means according to claim 4, **characterized in that** one memory (6) is formed by a read only memory.

6. The use of a floor cover sensor means according to claim 5, **characterized in that** the read only memory (6) includes an unambiguous identification.

7. The use of a floor cover sensor means according to any of claims 4 to 6, **characterized in that** the memory (7) is formed by a write/read memory.

8. The use of a floor cover sensor means according to any of claims 1 to 7, **characterized in that** at least one sensor (5) connected with the transmission means (3) is provided for measuring humidity.

9. The use of a floor cover sensor means according to any of claims 1 to 8, **characterized in that** at least one pressure sensor connected with the transmission means (3) is provided.

10. The use of a floor cover sensor means according to any of claims 1 to 9, **characterized in that** a temperature sensor (22) and if need be a humidity sensor are provided for measuring the temperature and if need be the humidity at the surface (23) of the floor cover.

11. The use of a floor cover sensor means according to any of claims 1 to 10, **characterized in that** a voltage supply (8) connected with the transmission means (3), in particular a battery, is provided.

12. The use of a floor cover sensor means according to any of claims 4 to 11, **characterized in that** the transmission means (3), the at least one temperature sensor (2), if need be the humidity sensor (5), if need be the at least one pressure sensor, and the at least one memory (6, 7) are formed by an integrated circuit.

13. A floor cover consisting of floor cover elements (10) for laying onto a floor screed (12), **characterized by** a floor cover sensor means (1) positioned at the underside (20) of at least one floor cover element (10) for sensing ambiance parameters of the floor covers, comprising at least one sensor (2) for measuring the temperature in the region of the floor cover, wherein said at least one temperature sensor (2) is provided to be positioned below the floor cover and is connected with means (3) for the wireless transmission of the measured temperature values to a receiver (14), the transmission means (3) being formed by a transponder and being also designed for receiving data.

14. The floor cover according to claim 13, **characterized in that** the floor cover sensor means (1) is positioned in a recess (19) at the underside (20) of a floor cover element (10).

15. The floor cover according to claim 14, **characterized in that** the floor cover sensor means (1) is glued with the underside (20) of the floor cover element (10).

16. The floor cover according to claim 14, **characterized in that** the floor cover sensor means (1) is detachably connected, for instance, screwed, to the underside (20) of the floor cover element (10).

17. The floor cover according to any of claims 13 to 16, **characterized in that** the floor cover elements (10) are made of wood.

18. The floor cover according to any of claims 13 to 17, **characterized in that** at least one element (10) comprises a bore (21) for accommodating a sensor (22) for measuring the temperature, and if need be for accommodating a sensor for measuring the humidity at the upper side (23) of the floor cover element (10).

19. A method of sensing ambiance parameters with floor covers, wherein the temperature is measured in the region of the floor cover, **characterized in that** the temperature values measured at the underside (20) of said floor cover, between the floor cover and the floor screed, are transmitted to means (3) designed as a transponder also for the receipt of data, for the wireless transmission of the measured temperature values, and are, on request, transmitted wirelessly from the transmission means (3) to a receiver (14).

20. The method according to claim 19, **characterized in that** humidity values measured at the underside (20) of the floor cover are transmitted wirelessly to the receiver (14).

21. The method according to claims 19 or 20, **characterized in that** pressure values measured at the underside (20) of the floor cover are transmitted wirelessly to the receiver (14).

22. The method according to any of claims 19 to 21, **characterized in that** temperature values measured at the upper side (23) of the floor cover are transmitted wirelessly to the receiver (14).

23. The method according to any of claims 19 to 22, **characterized in that** humidity values measured at the upper side (23) of the floor cover are transmitted wirelessly to the receiver (14).

24. The method according to any of claims 19 to 23, **characterized in that** data assigned to the floor cover are transmitted wirelessly to the receiver (14).

25. The method according to any of claims 19 to 24, **characterized in that** the temperature values, if need be the humidity values, if need be the pressure values, and if need be the assigned data are transmitted to the receiver (14) automatically at predetermined times.

26. The method according to any of claims 19 to 24, **characterized in that** the temperature values, if need be the humidity values, if need be the pressure values, and if need be the assigned data are transmitted to the receiver (14) on request.

27. The method according to any of claims 19 to 26, **characterized in that** the temperature values, if need be the humidity values, if need be the pressure values, and if need be the assigned data are automatically buffered in a memory (7) at predetermined times before being transmitted to the receiver (14).

28. The method according to any of claims 19 to 27, **characterized in that** the temperature values, if need be the humidity values, if need be the pressure values, and if need be the assigned data are encrypted before being transmitted to the receiver (14).

29. The method according to any of claims 19 to 28, **characterized in that** the temperature values and if need be humidity values are consulted for controlling the temperature of a floor heating arranged below the floor cover.

## Revendications

1. Utilisation d'un dispositif de saisie de revêtements de sol (1) pour saisir des paramètres concernant l'environnement de revêtements de sol avec au moins un capteur (2) pour mesurer la température dans la zone du revêtement de sol, **caractérisé en ce que** l'au moins un capteur de température (2) est prévu pour la mise en place entre le revêtement de sol et une chape, et est relié à un dispositif (3) pour la transmission sans fil des valeurs de température mesurées à un récepteur (14), lequel dispositif de transmission (3) est formé par un transpondeur et est également réalisé pour la réception de données.

2. Utilisation d'un dispositif de saisie de revêtements de sol (1) selon la revendication 1, **caractérisé en ce que** le dispositif de transmission (3) est formé par un système d'identification de haute fréquence (RFID).

3. Utilisation d'un dispositif de saisie de revêtements de sol selon la revendication 2, **caractérisé en ce que** le système d'identification de haute fréquence (RFID) est réalisé de manière à être passif.

4. Utilisation d'un dispositif de saisie de revêtements de sol selon l'une quelconque des revendications 1 à 3, **caractérisé en ce que** l'on prévoit au moins une mémoire (6, 7) en liaison avec le dispositif de transmission (3).

5. Utilisation d'un dispositif de saisie de revêtements de sol selon la revendication 4, **caractérisé en ce qu'**une mémoire (6) est formée par une mémoire morte.

6. Utilisation d'un dispositif de saisie de revêtements de sol selon la revendication 5, **caractérisé en ce que** la mémoire morte (6) contient une identification univoque.

7. Utilisation d'un dispositif de saisie de revêtements de sol selon l'une quelconque des revendications 4 à 6, **caractérisé en ce que** la mémoire (7) est formée par une mémoire à lecture/écriture.

8. Utilisation d'un dispositif de saisie de revêtements de sol selon l'une quelconque des revendications 1 à 7, **caractérisé en ce que** l'on prévoit au moins un capteur (5) en liaison avec le dispositif de transmission (3) pour la mesure de l'humidité.

9. Utilisation d'un dispositif de saisie de revêtements de sol selon l'une quelconque des revendications 1 à 8, **caractérisé en ce que** l'on prévoit au moins un capteur de pression en liaison avec le dispositif de transmission (3).

10. Utilisation d'un dispositif de saisie de revêtements de sol selon l'une quelconque des revendications 1 à 9, **caractérisé en ce qu'**un capteur de température (22) et le cas échéant un capteur d'humidité pour la mesure de la température et de l'humidité le cas échéant sont disposés à la surface (23) du revêtement de sol.

11. Utilisation d'un dispositif de saisie de revêtements de sol selon l'une quelconque des revendications 1 à 10, **caractérisé en ce que** l'on prévoit un dispositif d'alimentation en courant (8), en particulier une batterie, en liaison avec le dispositif de transmission (3).

12. Utilisation d'un dispositif de saisie de revêtements de sol selon l'une quelconque des revendications 4 à 11, **caractérisé en ce que** le dispositif de transmission (3), le capteur de température (2) au moins au nombre d'un, le capteur d'humidité (5) le cas échéant, le capteur de pression au moins au nombre d'un le cas échéant tout comme la mémoire (6, 7) au moins au nombre de une sont formés grâce à un circuit intégré.

13. Revêtement de sol se composant d'éléments de revêtement de sol (10) pour la pose sur une chape (12), **caractérisé par** un dispositif de saisie de revêtements de sol (1) disposé sur le dessous (20) d'au moins un élément de revêtement de sol (10), pour saisir des paramètres concernant l'environnement de revêtements de sol avec au moins un capteur (2) pour mesurer la température dans la zone du revêtement de sol, dans lequel l'au moins un capteur de température (2) est prévu pour la mise en place sous le revêtement de sol, et est relié à un dispositif (3) pour la transmission sans fil des valeurs de température mesurées à un récepteur (14), lequel dispositif de transmission (3) est formé par un transpondeur et est également réalisé pour la réception de données.

14. Revêtement de sol selon la revendication 13, caractérisé en ce le dispositif de saisie de revêtements de sol (1) est disposé dans un évidement (19) sur le dessous (20) d'un élément de revêtement de sol (10).

15. Revêtement de sol selon la revendication 14, **caractérisé en ce que** le dispositif de saisie de revêtements de sol (1) est collé avec le dessous (20) de l'élément de revêtement de sol (10).

16. Revêtement de sol selon la revendication 14, **caractérisé en ce que** le dispositif de saisie de revêtements de sol (1) est en liaison détachable avec le dessous (20) de l'élément de revêtement de sol (10), en étant vissé par exemple.

17. Revêtement de sol selon l'une quelconque des revendications 13 à 16, **caractérisé en ce que** les éléments de revêtement de sol (10) sont en bois.

18. Revêtement de sol selon l'une quelconque des revendications 13 à 17, **caractérisé en ce qu'**au moins un élément (10) présente une forure (21) pour accueillir un capteur (22) pour mesurer la température et pour accueillir le cas échéant un capteur pour mesurer l'humidité sur le dessus (23) de l'élément de revêtement de sol (10).

19. Procédé pour la saisie de paramètres concernant l'environnement pour des revêtements de sol, la température étant mesurée dans la zone du revêtement de sol, **caractérisé en ce que** les valeurs de température mesurées sur le dessous (20) du revêtement de sol, entre le revêtement de sol et la chape, sont retransmises à un dispositif (3) réalisé en tant que transpondeur également pour la réception de données pour la transmission sans fil des valeurs de température mesurées, et sont transmises sans fil par le dispositif de transmission (3), sur demande, à un récepteur (14).

20. Procédé selon la revendication 19, **caractérisé en ce que** des valeurs d'humidité mesurées sur le dessous (20) du revêtement de sol sont transmises sans fil au récepteur (14).

21. Procédé selon les revendications 19 ou 20, **caractérisé en ce que** des valeurs de pression mesurées sur le dessous (20) du revêtement de sol sont transmises sans fil au récepteur (14).

22. Procédé selon l'une quelconque des revendications 19 à 21, **caractérisé en ce que** des valeurs de température mesurées sur le dessus (23) du revêtement de sol sont transmises sans fil au récepteur (14).

23. Procédé selon l'une quelconque des revendications 19 à 22, **caractérisé en ce que** des valeurs d'humidité mesurées sur le dessus (23) du revêtement de sol sont transmises sans fil au récepteur (14).

24. Procédé selon l'une quelconque des revendications 19 à 23, **caractérisé en ce que** l'on transmet sans fil au récepteur (14) des données associées au revêtement de sol.

25. Procédé selon l'une quelconque des revendications 19 à 24, **caractérisé en ce que** les valeurs de température, les valeurs d'humidité le cas échéant, les valeurs de pression le cas échéant et les données associées le cas échéant sont transmises automatiquement à des moments prescrits au récepteur (14).

26. Procédé selon l'une quelconque des revendications 19 à 24, **caractérisé en ce que** l'on transmet sur commande au récepteur (14) les valeurs de température, les valeurs d'humidité le cas échéant, les valeurs de pression le cas échéant et les données associées le cas échéant.

27. Procédé selon l'une quelconque des revendications 19 à 26, **caractérisé en ce que** les valeurs de température, les valeurs d'humidité le cas échéant, les valeurs de pression le cas échéant et les données associées le cas échéant sont automatiquement stockées de manière intermédiaire à des moments prescrits dans une mémoire (7) avant la transmission au récepteur (14).

28. Procédé selon l'une quelconque des revendications 19 à 27, **caractérisé en ce que** les valeurs de température, les valeurs d'humidité le cas échéant, les valeurs de pression le cas échéant et les données associées le cas échéant sont codées avant la transmission au récepteur (14).

29. Procédé selon l'une quelconque des revendications 19 à 28, **caractérisé en ce que** les valeurs de température et les valeurs d'humidité le cas échéant sont exploitées pour réguler la température d'un système de chauffage par le sol situé sous le revêtement de sol.
